# FASCICULE DE BREVET EUROPEEN

(11) **EP 2 187 889 B1**
(45) Date de publication et mention de la délivrance du brevet: **06.11.2013**
(21) Numéro de dépôt: 08855583.4
(22) Date de dépôt: 12.09.2008
(51) Int. Cl.: A61K 31/52, C07D 473/16

(54) **UTILISATION DE DERIVES DE PURINE POUR LA FABRICATION D'UN MEDICAMENT**
VERWENDUNG VON PURINDERIVATEN ZUR HERSTELLUNG EINES MEDIKAMENTS
USE OF PURINE DERIVATIVES FOR THE MANUFACTURE OF A MEDICAMENT

(30) Priorité: 12.09.2007 FR 0706390
(43) Date de publication de la demande: 26.05.2010
(73) Titulaire: Centre National de la Recherche Scientifique, 75016 Paris (FR); Université de Rennes 1, 35065 Rennes Cedex (FR); UNIVERSITE PARIS DESCARTES, 75006 Paris (FR); Centre Hospitalier Universitaire de Brest, 29609 Brest Cedex (FR); Université de Bretagne Occidentale, 29238 Brest Cedex 3 (FR)
(72) Inventeur: MEIJER, Laurent, F-29680 Roscoff (FR); BETTAYEB, Karima, F-29680 Roscoff (FR); GALONS, Hervé, F-75014 Paris (FR); OUMATA, Nassima, F-75017 Paris (FR); BERTHOU, Christian, F-29200 Brest (FR); LESTER, Karine, F-29000 Quimper (FR)
(74) Mandataire: Le Coupanec, Pascale A.M.P.
(86) Numéro de dépôt international: PCT/FR2008/001278
(87) Numéro de publication internationale: WO 2009/068761

(56) Documents cités:
- WO-A1-98/05335
- WO-A2-03/022805
- SCHOW, STEVEN R. ET AL: "Synthesis and activity of 2,6,9-trisubstituted purines" BIOORGANIC & MEDICINAL CHEMISTRY LETTERS , 7(21), 2697-2702 CODEN: BMCLE8; ISSN: 0960-894X, 1997, XP002481170
- VESELY J ET AL: "INHIBITION OF CYCLIN-DEPENDENT KINASES BY PURINE ANALOGUES" EUROPEAN JOURNAL OF BIOCHEMISTRY, BERLIN, vol. 224, no. 2, 1 septembre 1994 (1994-09-01), pages 771-786, XP000576956 ISSN: 0014-2956

## Description

L'invention concerne l'utilisation de dérivés de purine pour la fabrication d'un médicament destiné au traitement des pathologies dans lesquelles un déséquilibre entre division cellulaire et apoptose est impliqué et plus particulièrement dans laquelle une apoptose excessive est à l'origine de la pathologie.

Elle concerne également certains de ces dérivés de purine.

Les pathologies dans lesquelles un déséquilibre entre division cellulaire et apoptose est impliqué sont en particulier la leucémie lymphoïde chronique et les maladies rénales telle que la polykystose.

La leucémie lymphoïde chronique, LLC, est un groupe hétérogène de maladies caractérisées par l'accumulation des cellules B monoclonales CD5+ dans le sang, la moelle osseuse et les organes hématopoïétiques.

Il s'agit d'une maladie dans laquelle les cellules B monoclonales ne subissent pas ou peu d'apoptose naturelle (mort cellulaire) et comportant un faible contingent de cellules B engagées dans le cycle cellulaire. En ce sens, cette maladie est une maladie quelque peu différente des maladies dans lesquelles on constate une prolifération excessive, qui n'a pas cessé, des cellules monoclonales, dans lesquelles les cellules monoclonales sont fortement engagées dans le cycle cellulaire et, dans lesquelles la résistance à l'apoptose (mort cellulaire) constitue un phénomène de pathogénécité secondaire.

La LLC est communément classée en catégories séparées incluant la leucémie lymphoïde chronique des lymphocytes B et la leucémie lymphoïde chronique des lymphocytes de type T. Par LLC, on entend communément leucémie lymphoïde chronique des lymphocytes B (LLCB).

La leucémie lymphoïde chronique de lignée B, appelée LLC-B, est une maladie du lymphocyte B responsable de l'accumulation de lymphocytes B de morphologie lymphocytique, exprimant des antigènes de membrane caractéristiques de la maladie, telles les molécules CD5 et CD23, dans le sang, entraînant une hyperlymphocytose, dans la moelle osseuse, entraînant une insuffisance médullaire, et dans les ganglions lymphatiques, entraînant une polydénopathie.

La LLC-B a été caractérisée comme une seule entité biologique avec une évolutivité variable.

La classification pronostique de Binet permet de fixer le profil évolutif de la maladie en trois stades A, B et C.

Parmi les sujets au stade A de la maladie, 41 % évolueront vers les stades B et C. Parmi les paramètres biologiques, le temps de doublement lymphocytaire inférieur à 6 mois, une élévation du taux de CD23 soluble ou de l'activité de la thymidine kinase sérique sont considérés de mauvais pronostic. Parmi les paramètres génétiques parfaitement identifiés de mauvais pronostic sont les formes non mutées de la maladie (gène de la chaîne lourde des immunoglobulines en position germinale en 14q32), les anomalies délétionnelles des chromosomes 11q, 17p ou les anomalies chromosomiques additionnelles de type 12q+. Les malades porteurs de LLC-B et exprimant ces caractères biologiques ont un temps de progression court : ainsi, 50% des malades non mutés ont évolué en 24 mois ; 50% des malades présentant un 17p-, un 11q- ou un 12q+ ont évolué à 15 mois. Les malades de stade A exprimant ces critères biologiques de gravité, les malades de stade B et C doivent bénéficier d'une attitude thérapeutique active.

Bien que les traitements actuels induisent des rémissions de la maladie, tous les malades rechutent et il existe actuellement un consensus pour dire que la LLC reste une maladie incurable.

La réelle question qui se pose aujourd'hui est de définir, dans les stades A de la maladie, les malades qui présentent un potentiel biologique d'évolutivité de gravité.

Le meilleur traitement de première ligne de la LLC-B reste à définir.

Les analogues des purines, en particulier la fludarabine, restent de loin les plus étudiés dans la LLC-B. La fludarabine seule induit un taux de réponse globale meilleur que l'utilisation des polychimiothérapies comportant des agents alkylants et une corticothérapie. La fludarabine induit plus de rémission complète hématologique (7 à 40%) que les polychimiothérapies de type CHOP, CAP (chloraminophène).

En dépit de la meilleure réponse observée avec la fludarabine le bénéfice observé sur la survie globale reste marginal. Des tentatives thérapeutiques actuelles vont vers les associations de fludarabine avec la chimiothérapie conventionnelle, par exemple fludarabine plus cyclophosphamide, notamment dans les formes réfractaires de la maladie. L'espoir de survie est seulement de 12 mois chez les patients réfractaires à la fludarabine. Ces combinaisons sont néanmoins douées d'une majoration de la toxicité du traitement, notamment hématologique.

Une infection est observée chez 50% des malades traités par une association de fludarabine et de cyclophosphamide. Un sepsis documenté ou une pneumopathie est observé pendant le traitement chez 25% des malades traités, une fièvre non documentée et/ou une hospitalisation chez 25% des autres.

Une révolution thérapeutique a été acquise par l'avènement des anticorps thérapeutiques. Dans la LLC-B, deux anticorps thérapeutiques émergent : le rituximab et l'alemtuzumab. Dans la LLC-B, l'activité du rituximab est handicapée par la faible expression de la cible, l'antigène CD20, sur le lymphocyte B de LLC. Le rituximab est développé dans la LLC-B en synergie avec les analogues de purines et/ou le cyclophosphamide (réponse globale de 59% observée avec l'association fludarabine-cyclophosphamide-rituximab chez des malades réfractaires à la fludarabine, dont seulement 5% de réponse complète).

L'activité de l'alemtuzumab, dirigée contre un antigène exprimé sur les leucocytes et les lymphocytes B leucémiques de LLC, avec une grande hétérogénéité de la densité membranaire de l'antigène, est handicapée par sa forte activité immunosuppressive et l'incidence importante des réactivations des infections cytomégaliques et d'infections opportunistes en cours ou après traitement : l'anticorps présente une forte activité immunosuppressive T. La réponse hématologique à l'alemtuzumab est de 33%, l'anticorps est capable de détruire les lymphocytes B clonaux dans le sang et la moelle osseuse, mais est peu opérant dans les ganglions lymphatiques. Ces points limitent l'utilisation de l'anticorps dans cette indication. La radioimmunothérapie par anti-CD20 couplé à l'yttrium-90 (Zévalin) induit un faible pourcentage de rémission dans la LLC-B et est responsable d'une importante myélosuppression.

Le brevet US 6812232 décrit des analogues de purine proches de ceux de l'invention pour leur activité d'inhibition de la prolifération cellulaire. Or, dans la LLC, la prolifération cellulaire excessive a cessé.

La demande de brevet WO2005/002584 propose, quant à elle, d'utiliser la Roscovitine, de préférence dans sa configuration absolue (R) dans le traitement de la leucémie lymphoïde chronique, et plus particulièrement de la leucémie lymphoïde chronique de lignée B.

La Roscovitine est une purine ayant la formule suivante:

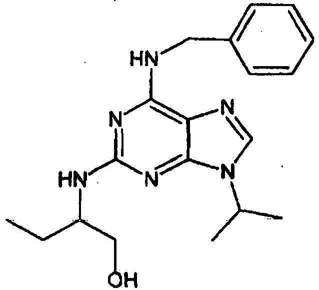

Or, on a maintenant découvert que des dérivés de la Roscovitine avaient une activité beaucoup plus élevée que la Roscovitine dans le traitement des pathologies dans lesquelles un déséquilibre entre division cellulaire et apoptose est impliqué et qu'ils avaient également dans certains cas une meilleurs solubilité que la Roscovitine.

Ainsi, l'invention décrit l'utilisation de composés de formule I suivante : 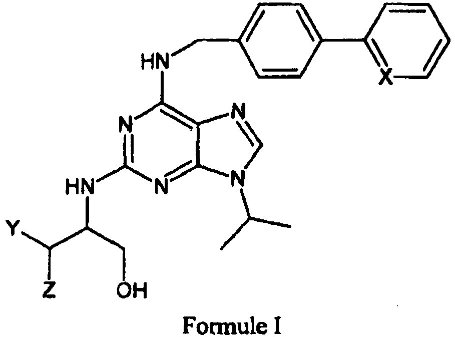 dans laquelle :
- X est C ou N,
- Y est CH₃ ou OH, et
- Z est H ou CH₃,
ou l'un de ses sels, hydrates, esters ou isomères pharmaceutiquement acceptables,
pour la fabrication d'un médicament destiné à traiter les pathologies dans lesquelles un déséquilibre entre division cellulaire et apoptose est impliqué.

Une des pathologies concernées est la leucémie lymphoïde chronique.

Plus particulièrement, la pathologie peut être la leucémie lymphoïde chronique de lignée B.

Une autre pathologie concernée est une maladie rénale, et plus particulièrement la polykystose.

Le sel oxalate des composés de formule I est un sel pouvant être utilisé dans la fabrication d'un médicament pour traiter les pathologies dans lesquelles un déséquilibre entre division cellulaire et apoptose est impliqué.

Les composés utilisés décrits dans l'invention à la formule I-I suivante correspondent à la formule I dans laquelle X est N :

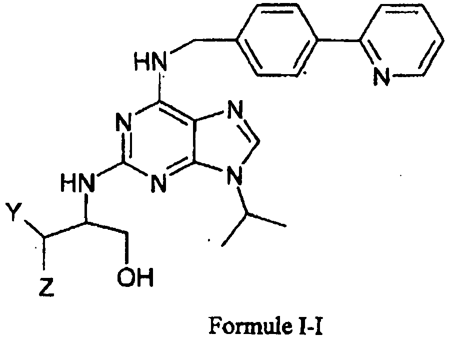

En effet, ces composés sont de 4 à 5 fois plus actifs dans les modèles cellulaires de la leucémie lymphoïde chronique et de la polykystose rénale et de 5 à 10 fois plus solubles en milieu aqueux que leurs correspondants dans lesquels X est C, comme on le verra ci-après.

Les composés utilisés décrits à la formule I-II suivante correspondent à la formule I dans laquelle X est N et Y est OH :

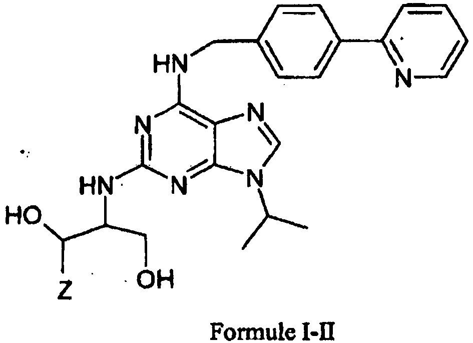

Les composés de structure I-II présentent une activité comparable à celle des dérivés I-I mais leur solubilité en milieu aqueux est encore augmentée.

Ainsi, la présente invention a pour objet, l'utilisation d'au moins un composé choisi parmi le composé de formule Id suivante : 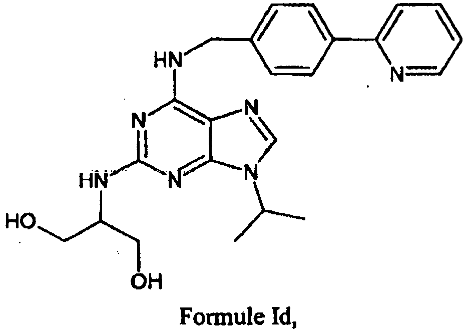 ou un de ses sels, hydrates, esters acylés choisis parmi les esters acétyle, nicotinyle et les esters d'aminoacides de série L ou de série D pharmaceutiquement acceptables,
le composé de formule Ie suivante : 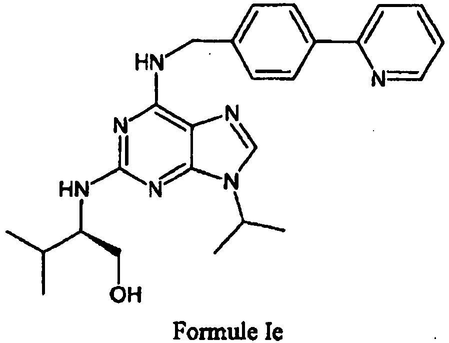 ou un de ses sels, hydrates, ou esters acylés choisis parmi les esters acétyle, nicotinyle et les esters d'aminoacides de série L ou de série D pharmaceutiquement acceptables,
le composé de formule Ig suivante : 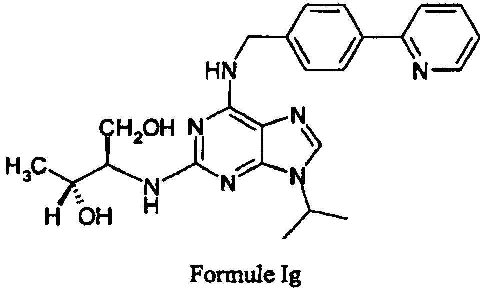 ou l'un des sels hydrate pharmaceutiquement acceptable, et
le composé de formule Ib suivante : 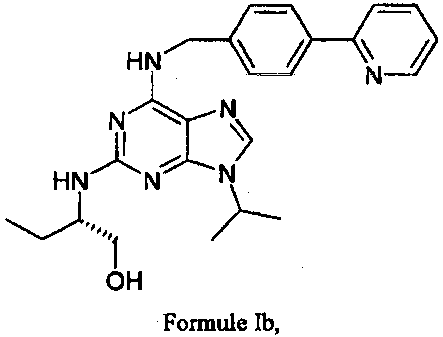 ou l'un de ses sels, hydrates ou esters acylés choisis parmi les esters acétyle, nicotinyle et les esters d'aminoacides de série L ou, de série D pharmaceutiquement acceptables,
pour la fabrication d'un médicament destiné à traiter la polykystose rénale.

Dans un mode de réalisation tout particulièrement préféré de l'invention, le composé a la configuration absolue (*S*). Ce composé a la formule Ib suivante : 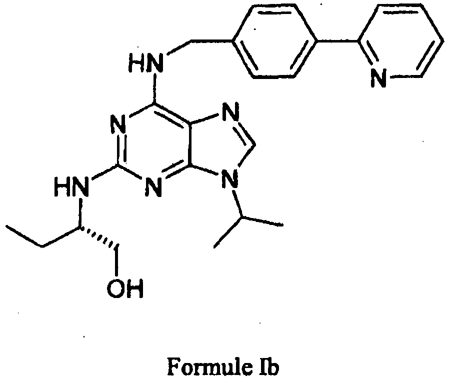 ou l'un de ses sels, hydrates ou esters acylés choisis parmi les esters acétyle, nicotinyle et les esters d'aminoacides de série L ou de série D pharmaceutiquement acceptables,

Ceci est particulièrement surprenant car, dans l'art antérieur, ce sont généralement les composés de configuration absolue (*R*) qui permettent d'obtenir les meilleurs résultats.

Dans un mode de réalisation tout particulièrement préféré de l'invention, le composé de formule Ib est sous la forme de son sel oxalate.

Le composé Id peut être obtenu, comme décrit à l'exemple 1 qui suit.

### Exemple 1 : Préparation du composé Id

Ce composé est obtenu en 3 étapes à partir de la dichloropurine selon le schéma suivant :

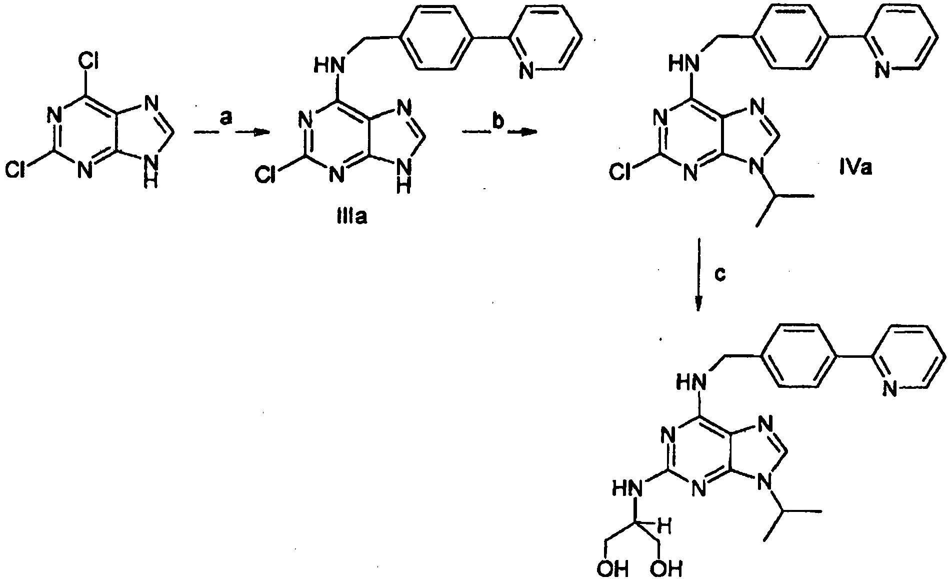

Réactifs et conditions :
a : 4-(2-pyridyl)benzylamine), n-BuOH, 110°C ; b : 2 bromopropane, K₂CO₃, DMSO ; c : Sérinol, chauffage 160 °C, 8h).

### Etape 1: 2-Chloro-6-[4-(2-pyridyl)phenylmethylamino)- purine (IIIa).

A une solution de 2,6-dichloropurine (2,3 g, 10 mmol) dans 20 mL de n-BuOH on ajoute (2,0 g, 1,05 mmol) de 4-(2-pyridylbenzylamine) et 3 mL de NEt₃. Après 3 h de chauffage à 110°C, le mélange est refroidi à 20°C et le solide est filtré et lavé par 5 mL de butanol froid puis séché sous vide. Rdt 85% PF> 250°C. ¹H NMR (DMSO_{d6}) d 4,80 (s, 2H, CH₂), 7,20(m, 1H, H_{pyridyl}); 7,45 (d, 2H, H_{phenyl}); 7,72(m, 2H, H_{pyridyl}); 7,95(m, 3H, H_{phenyl} et H-8), 8,54 (d, 1H, *J* = 4,8 Hz, H_{pyridyl}).

### Etape 2:

### 2-Chloro-9-isopropyl-6-[4-(2-pyridyl)phénylméthylamino]- purine (IVa).

A une solution de 2-chloro-6-[4-(2-pyridyl)phenylmethylamino]- purine (8 mmol) dans 10 mL de DMSO à 18-20 °C on ajoute K₂CO₃ (3,5 g, 24 mmol) et 1,9 mL (20 mmol) de 2-bromopropane. Après 5 h d'agitation à 18-20°C, on ajoute à nouveau du 2-bromopropane (0,5 mL) et l'agitation est poursuivie 5 h à 20°C. Après addition de 50 mL d'eau froide (5°C), le mélange est extrait par EtOAc (3x10 mL) et les phases organiques sont lavées par de la saumure (3x10 mL), séchées sur Na₂SO₄. Le dérivé IVa cristallise par évaporation du solvant. Il est trituré par 2mL de 2-propanol et filtré.

Rdt 86 % RMN-¹H (CDCl₃): δ 1,58 (d, 6H), 4,79 (hept, 1H), 4,85 (s large, 2H), 6,59 (s large 1H), 7,20-7,23 (m, 1H), 7,49 (d, 2H), 7,73-7,71 (m, 2H), 7,79 (s, 1H), 7,98 (d, 2H), 8,71 (d, 1H).

### Etape 3: 2-(1,3-Dihydroxyprop-2-ylamino)-6-[4-(2-pyridyl)phénylméthylamino]-9-iso-propylpurine). Id

Un mélange du composé IVa (10 mmol) et de sérinol (2-aminopropane-1,3-diol) (2 mL) est chauffé sous N₂, à 160°C pendant 8 h. Après refroidissement à 20°C, on ajoute 20 mL d'eau et le mélange est extrait par EtOAc (4x10 mL). La solution organique est lavée par 2x 20 mL d'eau, séchée et évaporée. Le dérivé Id cristallise par trituration avec Et₂O.

PF 114-117 °C Rdt 74 %. RMN-¹H (CDCl₃) 8: 1,52(d, 6H); 3,78(m, 4H) ; 3,96(m, 1H); 4,55(hept, 1H); 4,76(s, 2H); 5,40(s,1H); 6,20(s, 1H); 7,12(m, 1H); 7,38(d, 2H); 7,48(s, 1H); 7,62(m, 2H); 7,90(d, 2H); 8,60(d, 1H).

Selon un mode de réalisation préféré de l'invention, le composé utilisé est le sel oxalate du composé de formule Ie. Ce composé a la formule If suivante:

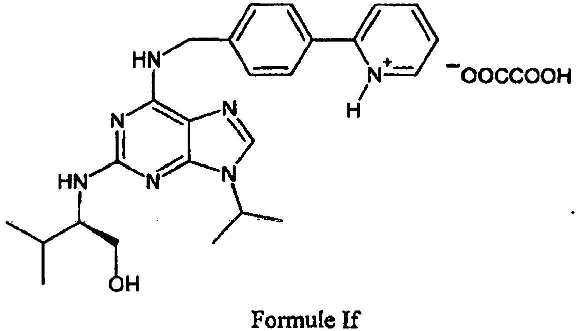

Il doit être noté que l'un des azotes de la purine peut être impliqué dans la formation du sel qui correspond à l'association d'une molécule de la purine avec le diacide. Le composé 1g est le (1R,2R)-**(Dihydroxyl1,3-but-2-ylamino)-6-[4-(2-pyridyl)phénylméthylamino)-9-iso-propylpurine). Ig**

Il est obtenu comme le produit Id mais en remplaçant dans la dernière étape l'aminopropanediol par le L-thréoninol ou (1R-2R)-2-aminobutan-1,3-diol.

Il a les caractéristiques mesurées par RMN suivantes :
RMN : 1,2 (d, 3H); 1,4(d, 6H); 3,70(m, 4H) ; 4,10(m, 1H) 4,52(hept, 1H); 4,72(s large, 2H); 5,50(s, 1H); 6,2 (s large, 1H); 7,15(m, 1H); 7,4(d, 2H); 7,42(s, 1H); 7,55(m, 2H); 7,85(d, 2H); 8,60(d, 1H).

Selon un mode de réalisation préféré de l'invention, le composé utilisé est le composé de formule Ih suivante :

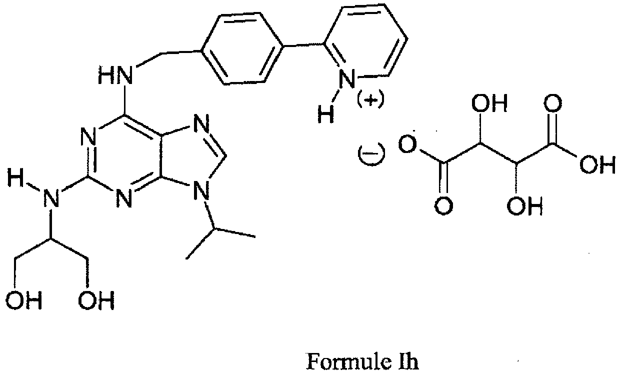

Il doit être noté que l'un des azotes de la purine peut être impliqué dans la formation du sel qui correspond à l'association d'une molécule de la purine avec le diacide. Les trois isomères de l'acide tartrique peuvent être utilisés.

Ce composé est le tartrate de 2-(1,3-dihydroxyprop-2-ylamino)-6-[4-(2-pyridyl)phénylméthylamino]-9-iso-propylpurine (Ih).

Il est obtenu de la façon suivante.

A une solution de 2 mmol de **Id** en solution dans l'*iso*-propanol (1mL) porté à 70-80°C on ajoute 2,1 mmol d'acide tartrique dissout dans l'*iso*-propanol (1mL). Après refroidissement on isole par filtration le tartrate de **Id.**

Les sels pharmaceutiquement acceptables peuvent avantageusement être choisis parmi un sel oxalate, tartrate, chlorhydrate et fumarate.

En particulier, les esters des composés de formule I sont également décrits dans l'invention.

Des esters des composés de formule I décrits sont par exemple les esters acylés tels que les esters acétyle, nicotinyle et les esters d'aminoacides de série L ou de série D.

D'autres esters sont formés à partir d'aminoacides tels que la valine ou la leucine.

Les esters particulièrement préférés ont les formules II-1 et II-3 suivantes, et sont objets de l'invention :

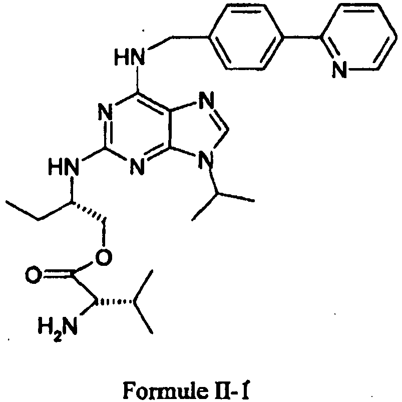

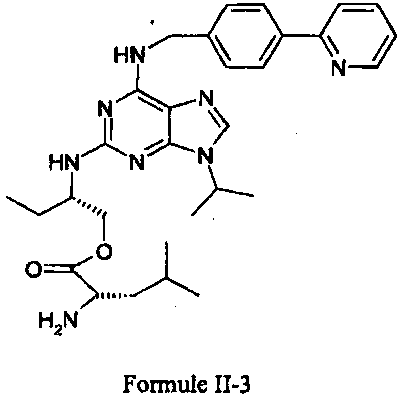

En effet, ces esters sont des précurseurs (prodrugs) des produits de formule Ib.

Leur utilisation dans la fabrication d'un médicament destiné à traiter les pathologies dans lesquelles un déséquilibre entre division cellulaire et apoptose est impliqué est également décrit dans l'invention.

L'invention sera mieux comprise et d'autres caractéristiques et avantages de celle-ci apparaîtront plus clairement à la lecture de la description explicative qui suit et qui est faite en référence aux figures dans lesquelles :
- la figure 1 jointe montre l'induction de la mort cellulaire par le composé de formule Ia, et le sel oxalate du composé de formule Ia dans des cellules de leucémie lymphoïde chronique, en comparaison à l'induction de la mort cellulaire induite par la Roscovitine,
- la figure 2 montre l'induction de la mort cellulaire par les composés de formule Ie et If dans les cellules de leucémie lymphoïde chronique.

Les effets de la Roscovitine et du composé de formule Ia, de son sel oxalate ont été testés à diverses concentrations, dans des dosages de kinase.

Le composé Ia présente la formule suivante :

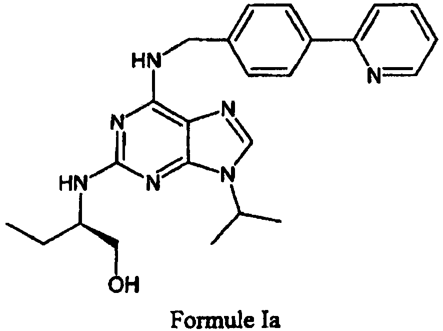

Ces tests ont été effectués comme suit :

### Tampons

*tampon A:* 10 mM MgCl₂, 1 mM EGTA, 1 mM DTT, 25 mM Tris-HCl pH 7.5, 50 µg héparine/ml.

*tampon C:* 60 mM glycérophosphate, 15 mM p-nitrophénylphosphate, 25 mM MOPS (pH 7.2), 5 mM EGTA, 15 mM MgCl₂, 1 mM DTT, 1 mM sodium vanadate, 1 mM phénylphosphate.

### Préparation et dosage des kinases

Les activités kinases ont été dosées dans le tampon A ou C, à 30 °C, et une concentration finale d'ATP de 15 µM. Des contrôles sont effectués avec des dilutions appropriées de diméthylsulfoxide.

*CDK1*/*cyclin B* (ovocytes en phase M d'étoile de mer, native) et *CDK5*/*p25* (humaine, recombinante) ont été préparés et dosés comme décrit dans Leclerc S. et al., J Biol Chem 2001;276:251-60.). Le dosage est effectué avec 1 mg histone H1/ml, en présence de 15 µM [γ-³³P] ATP (3,000 Ci/mmole; 10 mCi/ml) dans un volume final de 30 µl. Après 30 min. d'incubation à 30°C, des aliquots de 25 µl de surnageant ont été déposés sur des papiers 2.5 x 3 cm pièces de phosphocellulose Whatman P8, puis, 20 sec. plus tard, les filtres ont été lavés 5 fois dans une solution de 10 ml acide phosphorique/litre. Les filtres sont ensuite comptés en présence de 1 ml ACS (Amersham)

*CDK2*/*cyclin A et CDK2*/*cyclin E* (humain, recombinante, exprimées en cellules d'insecte) sont dosées comme CDK1.

*CDK9*/*cyclin T* (humain, recombinante, exprimées en cellules d'insecte) est dosée comme CDK1/cyclin B, avec un fragment pRB (AA773-928) (3.5 µg/dosage) comme substrat.

*GSK-3*α/β (cerveau de porc, native, purifiée par affinité) est dosée comme CDK1/cyclin B, mais dans le tampon A et avec un substrat GSK-3 spécifique (GS-1: YRRAAVPPSPSLSRHSSPHQSpEDEEE) (Bach S. et al., J Biol Chem 2005; 280: 31208-19).

*CK1δ*/*e* (cerveau de porc, native, purifiée par affinité) est dosée comme CDK1/cyclin B, mais dans le tampon A et avec un substrat spécifique, RRKHAAIGSpAYSTTA (Reinhardt J. et al. Protein Expr & Purif 2007;54:101-9).

DYRK1A (humain, recombinante, exprimées en cellules E. coli) est dosée comme CDK1/cyclin B.

Les valeurs de concentrations inhibitrices moyennes CI₅₀ ont été calculées à partir des courbes dose-réponse et sont reportées en µM dans le tableau I suivant:

**Tableau I**

| **Kinase** | **(*R*)-Roscovitine** | **Composé de formule Ia Forme (*R*)** | **Composé de formule Ib Forme (*S*)** |
|---|---|---|---|
| CDK1/cycline B | 0.33 | 0.09 | 0.15 |
| CDK2/cycline A | 0.21 | 0.072 | 0.080 |
| CDK2/cycline E | 0.17 | 0.041 | 0.060 |
| CDK5/p25 | 0.28 | 0.11 | 0.12 |
| CDK7/cycline H | 0.80 | 1.10 | - |
| CDK9/cycline T | 0.23 | 0.18 | 0.11 |
| CK1 | 4.00 | 0.40 | 0.61 |
| DYRK1A | 3.00 | 3.60 | 0.9 |
| Erk2 | 11.00 | 3.60 | 2.1 |
| GSK-3α/β | 60.00 | 12.0 | ≥ 30.00 |

On voit à partir du tableau I que pour toutes les protéines kinases, le composé de formule Ib de configuration (S) présente des activités inhibitrices des différentes kinases qui sont proches des activités de la Roscovitine et qui sont légèrement inférieures aux activités du composé de formule Ia, c'est-à-dire de son homologue de configurations absolue *(R).*

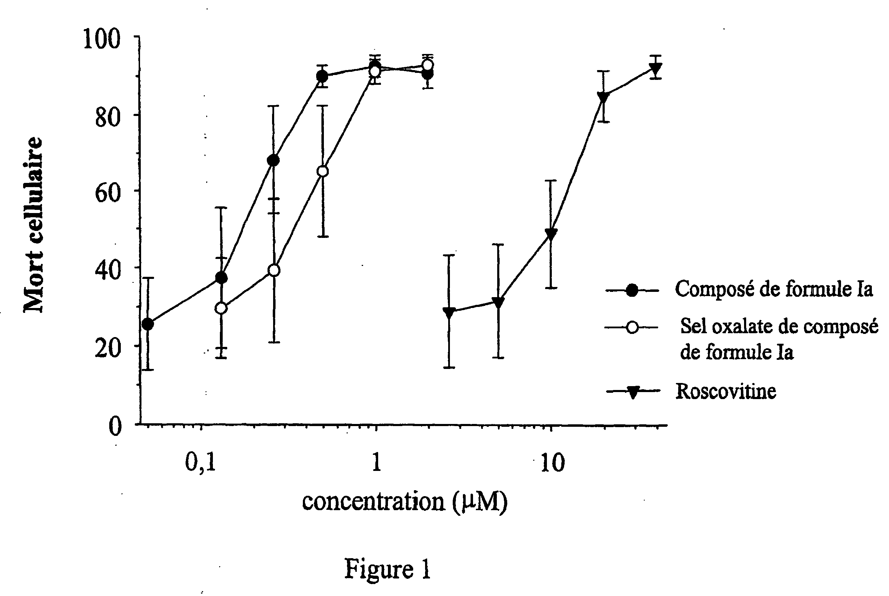

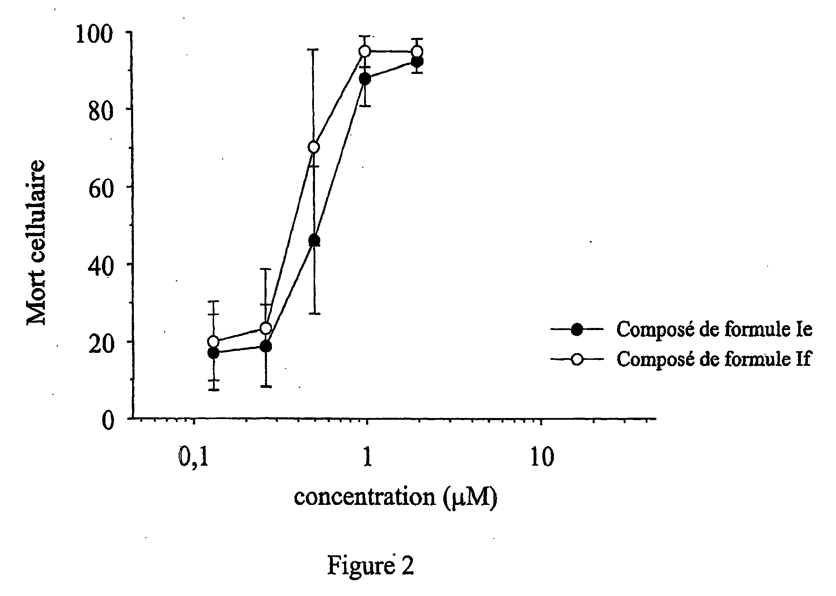

Cependant, lorsque le composé de formule Ia, ainsi que son sel oxalate et des composés de formule Ie et If, ont été testés sur des cellules de leucémie lymphoïde chronique de lignée B prélevées chez des patients ayant ce type de leucémie lymphoïde chronique, on constate que, de façon surprenante, ces composés ont une activité inductrice de l'apoptose sur les cellules de la LLC qui sont très supérieures, de 50 à 100 fois supérieure, à l'activité de la Roscovitine, comme on le voit en figures 1 et 2.

De plus, l'effet des composés Ia, Ib, Ic, Id, Ie et If et du sel oxalate du composé Ia sur l'induction de la mort cellulaire des lymphocytes B2 provenant de patients a été comparé à l'effet de la (R) Roscovitine sur cette même induction.

Le composé Ic présente la formule suivante :

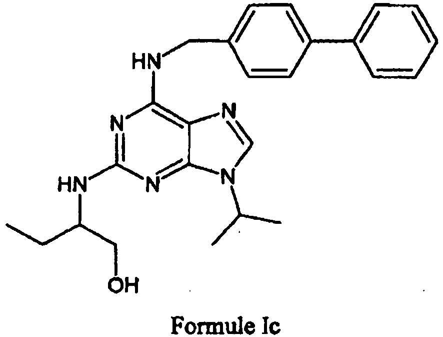

Les lymphocytes B2 sont les lymphocytes impliqués dans la leucémie lymphoïde chronique de lignée B2.

La viabilité cellulaire est déterminée par la réduction de la 3-(4,5-diméthylthiazol-2-yl)-5-(3-carboxyméthoxyphènyl)-2-(4-sulfophènyl)-2/H-tétrazolium (MTS).

La mort cellulaire est déterminée par la mesure du niveau de l'activité de la lactate déhydrogénase (LDH) libérée lors de la lyse des cellules. Les deux procédés sont décrits en détail dans "Ribas J, Boix J. Cell differentiation, caspase inhibition, and macromolecular synthesis blockage, but not BCL-2 or BCL-XL proteins protect SH-SY5Y cells from apoptosis triggered by two CDK inhibitory drugs. Exp. Cell Res. 2004; 295, 9-24".

Les résultats obtenus sont indiqués au tableau 2 suivant :

**Tableau 2**

| **Composé de formule** | **Induction de la mort cellulaire** |
|---|---|
| (R)-roscovitine | 8,96 |
| la [référence] | 0,20 |
| Ib | 0,09 |
| Oxalate de Ia | 0,2 |
| Ic | 1,25 |
| Id | 0,25 |
| Ie | 0,51 |
| If | 0,38 |

Dans le tableau 2, les valeurs indiquées sont les valeurs de concentrations moyennes inhibitrices, CI50, exprimées en µM.

L'effet des composés Ib et Id de l'invention sur la polykystose rénale a également été testé sur la lignée MDCK en comparaison avec la (R) Roscovitine. Les composés Ib et Id sont 50 à 60 fois plus actif que la Roscovitine.

La solubilité des composés de l'invention dans lesquels X est N, on l'a dit, sont 5 à 10 fois plus solubles dans l'eau que ceux dans lesquels X est C:
Ainsi, le composé de formule Ic, dans lequel X est C, a une solubilité dans l'eau de 0,5 µg/mL alors que le composé correspondant dans lequel X est N, c'est-à-dire le composé de formule Ib, a une solubilité dans l'eau de 3,3 µg/mL.

Les composés de formule Ib et Id à Ih *per se* sont également des objets de l'invention.

Ainsi, les composés de l'invention sont particulièrement efficaces pour une utilisation dans la fabrication d'un médicament pour traiter les maladies rénales, et en particulier la polykystose. De la même façon, ils sont particulièrement appropriés pour une administration dans une méthode de traitement de patients affectés d'une maladie rénale, et en particulier d'une polykystose.

L'homme du métier comprendra aisément que les composés de l'invention peuvent être utilisés en mélange entre eux, de deux ou plus, et également en association avec d'autres composés ayant une activité thérapeutique dans le traitement des maladies rénales telle que la polykystose, et/ou en association avec tout excipient pharmaceutiquement acceptable pour la fabrication d'un médicament et que ces associations et mélanges font également partie de l'invention.

## Revendications

1. Utilisation d'au moins un composé choisi parmi le composé de formule Id suivante : 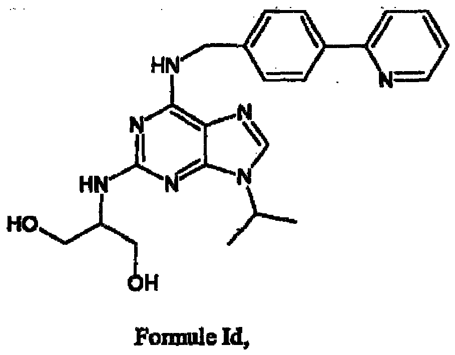 ou un de ses sels, hydrates, esters acylés choisis parmi les esters acétyle, nicotinyle et les esters d'aminoacides de série L ou de série D pharmaceutiquement acceptables,
le composé de formule le suivante : 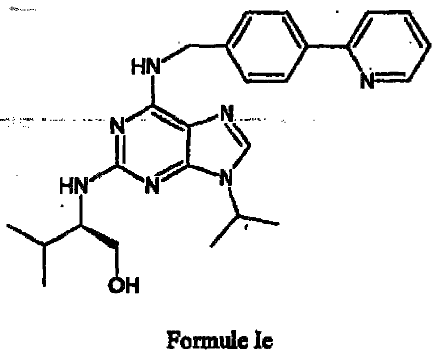 ou un de ses sels, hydrates, ou esters acylés choisis parmi les esters acétyle, nicotinyle et les esters d'aminoacides de série L ou de série D pharmaceutiquement acceptables,
le composé de formule **Ig** suivante : 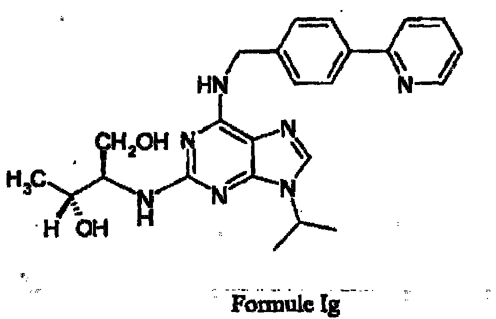 ou l'un de ses sels ou hydrates pharmaceutiquement acceptable, et
le composé de formule Ib suivante : 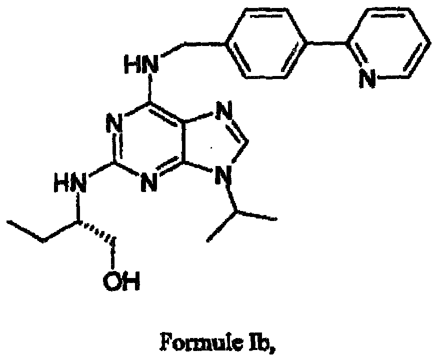 ou l'un de ses sels, hydrates ou esters acylés choisis parmi les esters acétyle, nicotinyle et les esters d'aminoacides de série L ou de série D pharmaceutiquement acceptables,
pour la fabrication d'un médicament destiné à traiter la polykystose rénale.

2. Utilisation selon la revendication 1, **caractérisée en ce que** le sel pharmaceutiquement acceptable est un sel oxalate ou tartrate, ou chlorhydrate ou fumarate.

3. Utilisation selon l'une des revendications 1 ou 2, **caractérisée en ce que** le au moins un composé est le composé de formule Ib suivante : 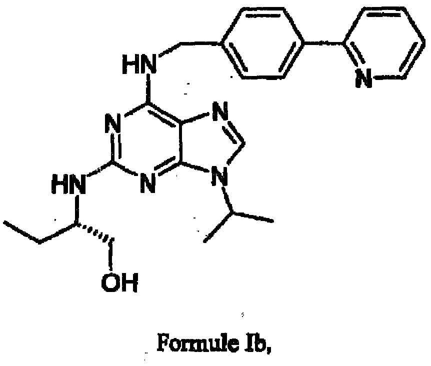 ou l'un de ses sels, hydrates ou esters acylés choisis parmi les esters acétyle, nicotinyle et les esters d'aminoacides de série L ou de série D pharmaceutiquement acceptables.

4. Utilisation selon la revendication 3, **caractérisée en ce que** le au moins un composé est le sel oxalate du composé de formule Ib.

5. Composé de formule Ib suivante : 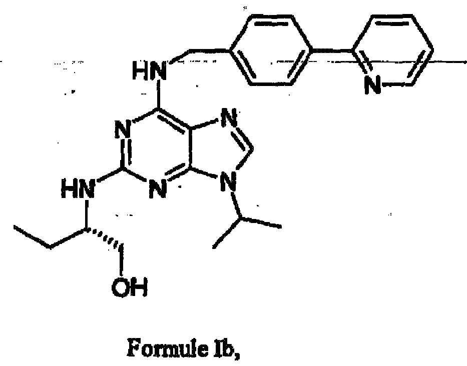 ou un de ses sels, hydrates, ou esters acylés choisis parmi les esters acétyle, nicotinyle et les esters d'aminoacides de série L ou de série D.

6. Composé de formule Id suivante : 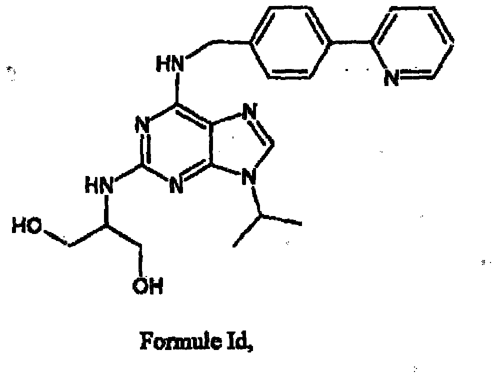 ou un de ses sels hydrates ou esters acylés choisis parmi les esters acétyle, nicotinyle et les esters d'aminoacides de série L ou de serie D,
ou de formule Ie suivante : 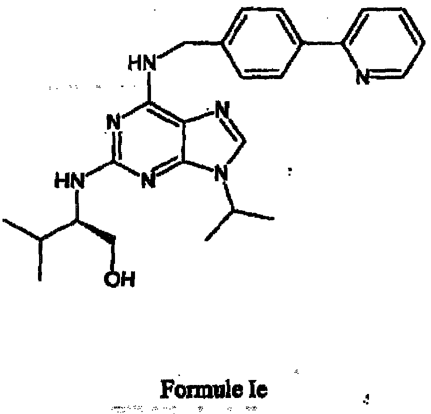 ou les sels, hydrates, ou esters acylés choisis parmi les esters acétyle, nicotinyle et les esters d'aminoacides de série L ou de série D de celui-ci, ou
de formule If suivante: 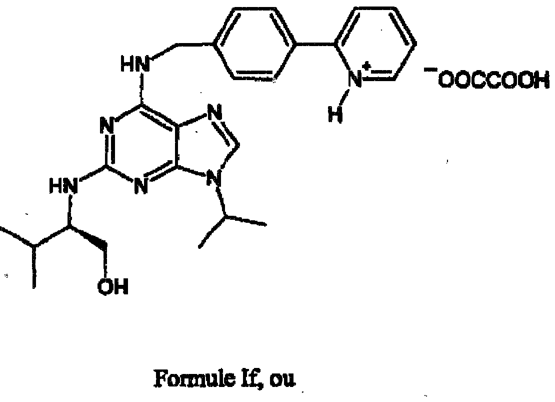 de formule Ig suivante : 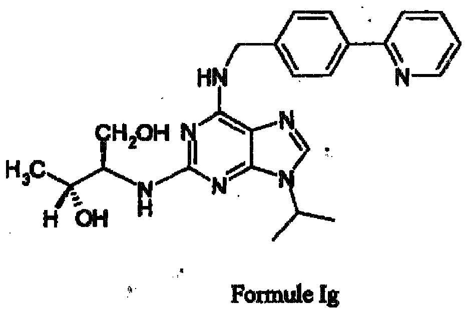 ou un de ses sels, hydrates, esters acylés choisis parmi les esters acétyle, nicotinyle et les esters d'aminoacides de série L où de série D, ou
de formule Ih suivante :

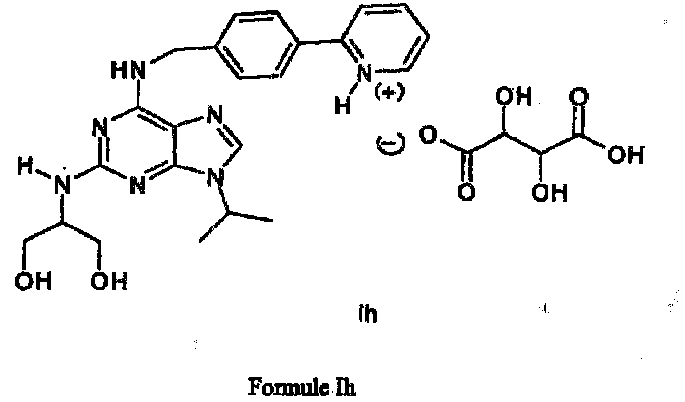

7. Composés **caractérisés en ce qu'**ils ont les formules II-I et II-3 suivantes :

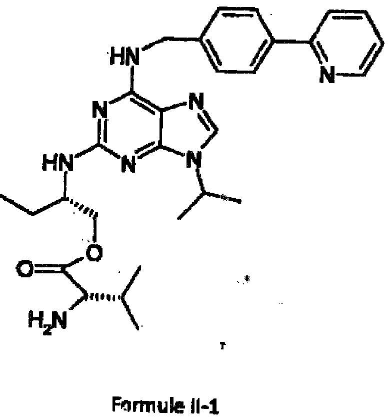

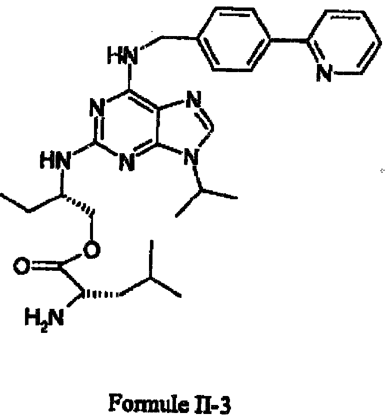

## Patentansprüche

1. Verwendung mindestens einer Verbindung, ausgewählt aus der Verbindung der folgenden Formel Id 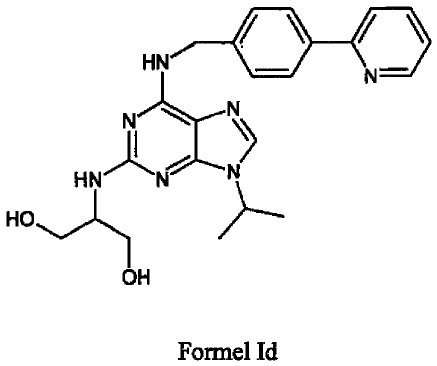 oder eines ihrer pharmazeutisch akzeptablen Salze, Hydrate, oder acylierten Ester, ausgewählt aus Acetylestern, Nicotinylestern und Estern von Aminosäuren der L-Reihe oder D-Reihe,
der Verbindung der folgenden Formel Ie: 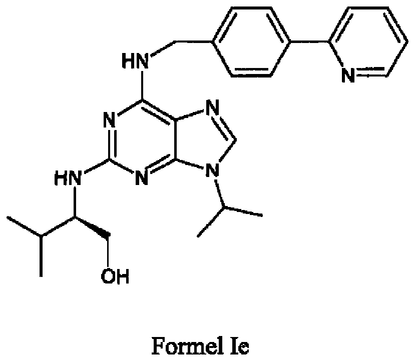 oder eines ihrer pharmazeutisch akzeptablen Salze, Hydrate, oder acylierten Ester, ausgewählt aus Acetylestern, Nicotinylestern und Estern von Aminosäuren der L-Reihe oder D-Reihe,
der Verbindung der Formel Ig: 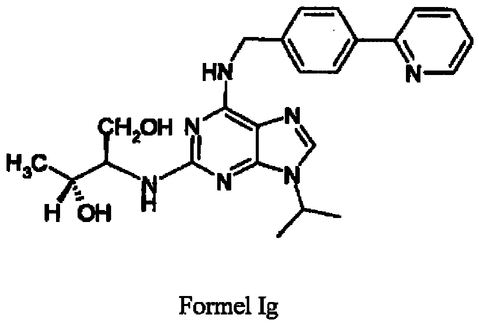 oder einer ihrer pharmazeutisch akzeptablen Salze oder Hydrate, und
der Verbindung der folgenden Formel Ib: 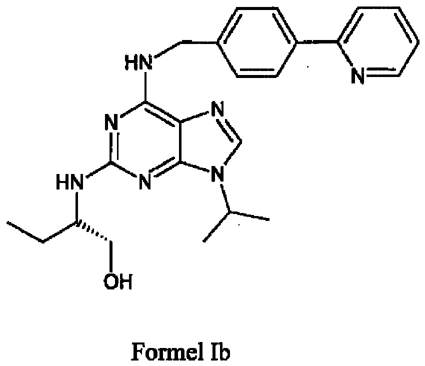 oder eines ihrer pharmazeutisch akzeptablen Salze, Hydrate, oder acylierten Ester, ausgewählt aus Acetylestern, Nicotinylestern und Estern von Aminosäuren der L-Reihe oder D-Reihe,
zur Herstellung eines Arzneimittels für die Behandlung der polyzystischen Nierenerkrankung.

2. Verwendung nach Anspruch 1, **dadurch gekennzeichnet, dass** das pharmazeutisch akzeptable Salz ein Oxalatsalz oder Tartratsalz oder ein Chlorhydrat oder Fumarat ist.

3. Verwendung nach einem der Ansprüche 1 oder 2, **dadurch gekennzeichnet, dass** mindestens eine Verbindung die Verbindung der folgenden Formel Ib ist: 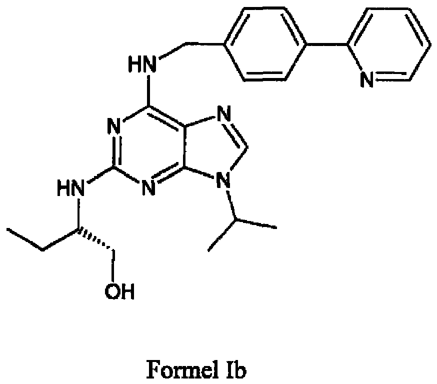 oder eines ihrer pharmazeutisch akzeptablen Salze, Hydrate, oder acylierten Ester, ausgewählt aus Acetylestern, Nicotinylestern und Estern von Aminosäuren der L-Reihe oder D-Reihe.

4. Verwendung nach Anspruch 3, **dadurch gekennzeichnet, dass** die mindestens eine Verbindung das Oxalatsalz der Verbindung der Formel Ib ist.

5. Verbindung der folgenden Formel Ib: 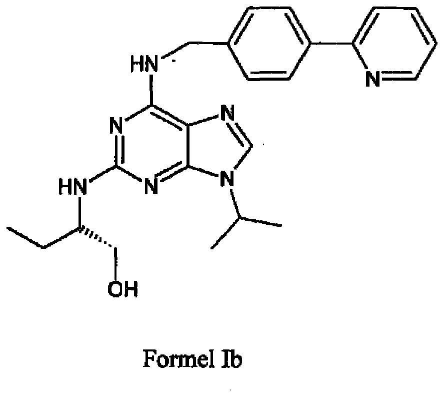 oder eines ihrer pharmazeutisch akzeptablen Salze, Hydrate, oder acylierten Ester, ausgewählt aus Acetylestern, Nicotinylestern und Estern von Aminosäuren der L-Reihe oder D-Reihe.

6. Verbindung der folgenden Formel Id: 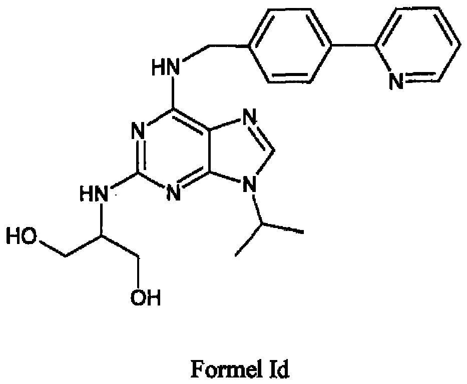 oder eines ihrer pharmazeutisch akzeptablen Salze, Hydrate, oder acylierten Ester, ausgewählt aus Acetylestern, Nicotinylestern und Estern von Aminosäuren der L-Reihe oder D-Reihe,
oder der folgenden Formel Ie: 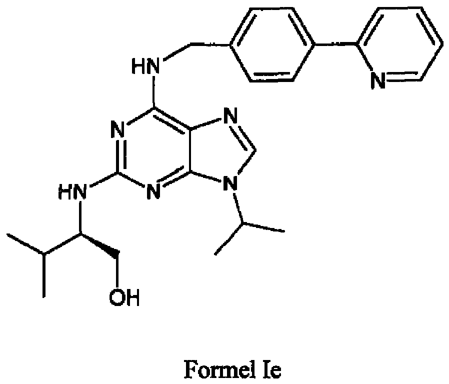 oder eines ihrer pharmazeutisch akzeptablen Salze, Hydrate, oder acylierten Ester, ausgewählt aus Acetylestern, Nicotinylestern und Estern von Aminosäuren der L-Reihe oder D-Reihe, oder
der folgenden Formel If: 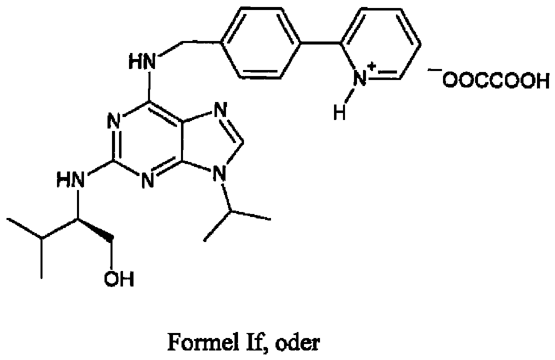 der folgenden Formel Ig: 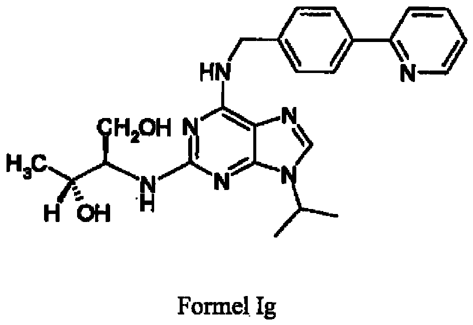 oder eines ihrer pharmazeutisch akzeptablen Salze, Hydrate, oder acylierten Ester, ausgewählt aus Acetylestern, Nicotinylestern und Estern von Aminosäuren der L-Reihe oder D-Reihe, oder
der folgenden Formel Ih:

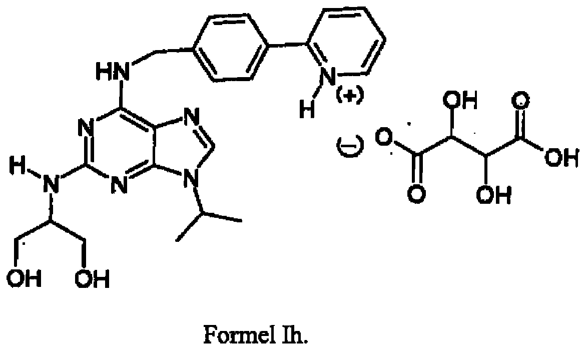

7. Verbindungen, **dadurch gekennzeichnet, dass** sie die nachfolgenden Formeln II-1 und II-3 aufweisen:

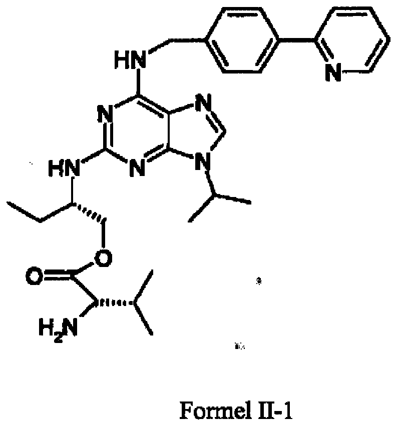

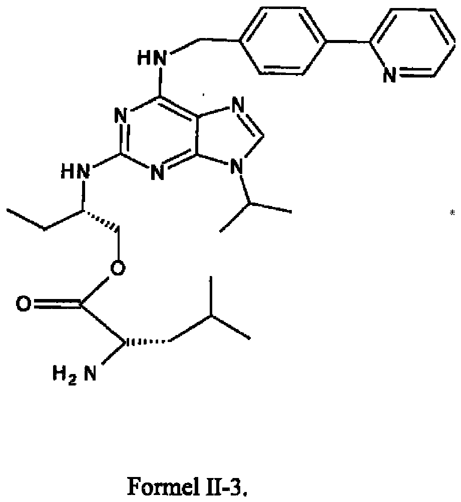

## Claims

1. Use of at least one compound chosen from the compound of following formula Id: 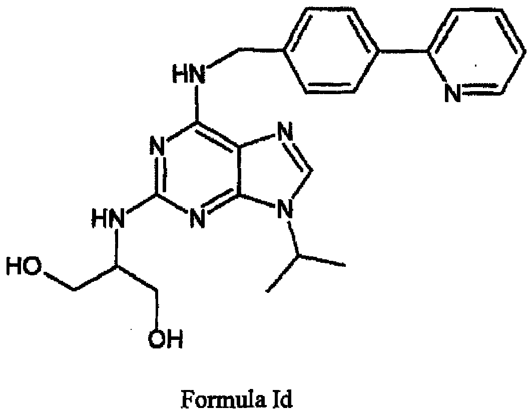 or one of its pharmaceutically acceptable salts, hydrates, acyl esters chosen from acetyl esters, nicotinyl esters and esters of amino acids of the L series or D series,
the compound of following formula Ie: 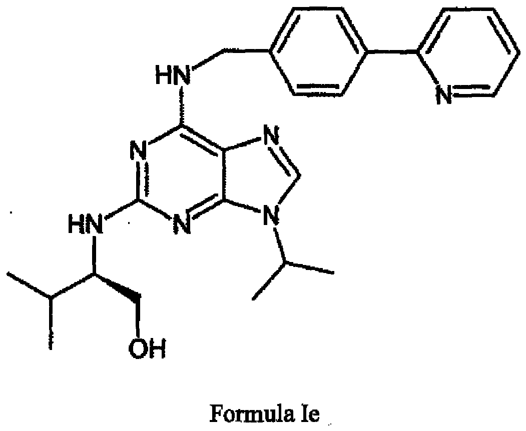 or one of its pharmaceutically acceptable salts, hydrates or acyl esters chosen from acetyl esters, nicotinyl esters and esters of amino acids of the L series or D series,
the compound of the following formula Ig: 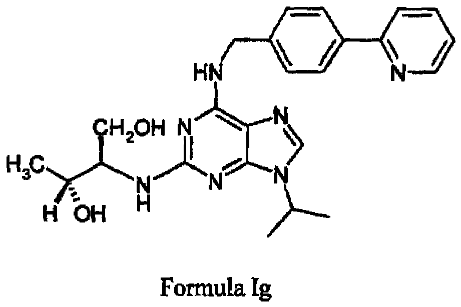 or one of its pharmaceutically acceptable salts or hydrates, and
the compound of following formula Ib: 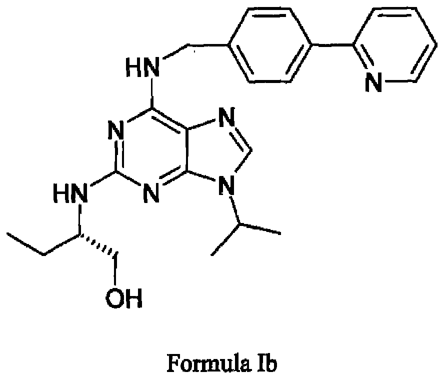 or one of its pharmaceutically acceptable salts, hydrates or acyl esters chosen from acetyl esters, nicotinyl esters and esters of amino acids of the L series or D series,
for the manufacture of a medicament intended to treat polycystic kidney disease.

2. Use according to claim 1, wherein the pharmaceutically acceptable salt is an oxalate or tartrate or hydrochloride or fumarate salt.

3. Use according to claim 1 or 2, wherein the at least one compound is the compound of following formula Ib: 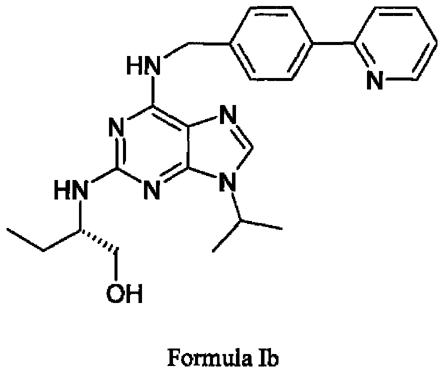 or one of its pharmaceutically acceptable salts, hydrates or acyl esters chosen from acetyl esters, nicotinyl esters and esters of amino acids of the L series or D series.

4. Use according to claim 3, wherein the at least one compound is the oxalate salt of the compound of formula Ib.

5. The compound of following formula Ib: 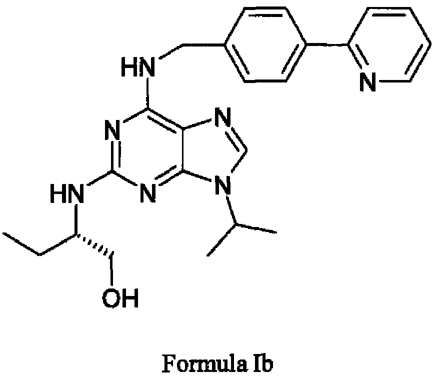 or one of its salts, hydrates or acyl esters chosen from acetyl esters, nicotinyl esters and esters of amino acids of the L series or D series.

6. The compound of following formula Id: 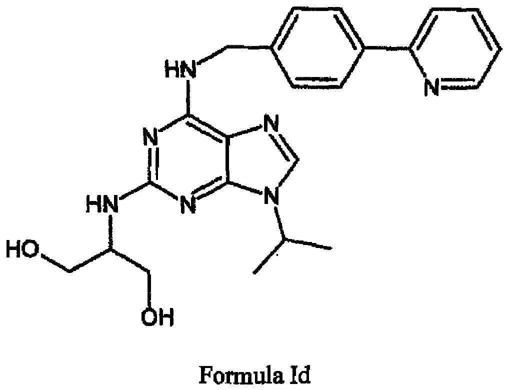 or one of its salts, hydrates or acyl esters chosen from acetyl esters, nicotinyl esters and esters of amino acids of the L series or D series,
or of following formula Ie: 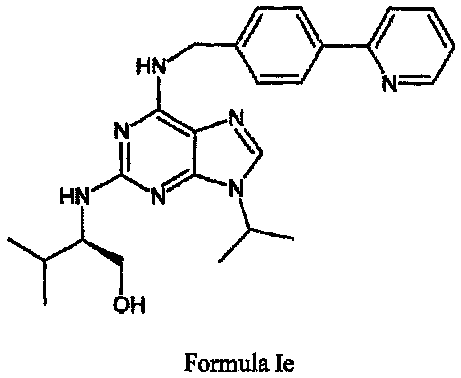 or one of its salts, hydrates or acyl esters chosen from acetyl esters, nicotinyl esters and esters of amino acids of the L series or D series,
or of following formula If: 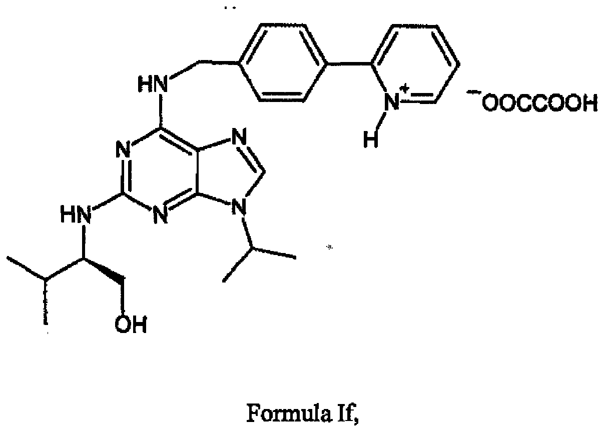 or of following formula Ig: 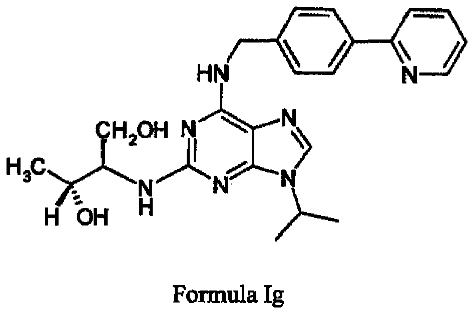 or one of its salts, hydrates or acyl esters chosen from acetyl esters, nicotinyl esters and esters of amino acids of the L series or D series,
or of following formula Ih:

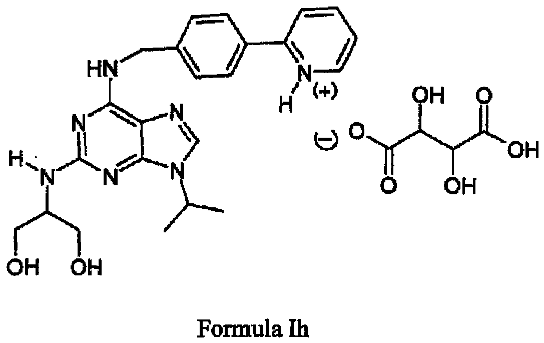

7. Compounds, **characterized in that** they have the following formulae II-1 and II-3:
